# EUROPÄISCHE PATENTSCHRIFT

(11) **EP 4 198 002 B1**
(45) Veröffentlichungstag und Bekanntmachung des Hinweises auf die Patenterteilung: **24.07.2024**
(21) Anmeldenummer: 21215373.8
(22) Anmeldetag: 17.12.2021
(51) Int. Cl.: C07C 45/00, C07F 15/00

(54) **PT-XANTHEN-IOD-KOMPLEX**
PT-XANTHENE-IODINE COMPLEX
COMPLEXE PT-XANTHÈNE-IODE

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- WO-A2-2010/129030
- PETOCZ G ET AL: "Xantphos as cis- and trans-chelating ligand in square-planar platinum(II) complexes. Hydroformylation of styrene with platinum-xantphos-tin(II)chloride system", JOURNAL OF ORGANOMETALLIC CHEMISTRY, ELSEVIER, AMSTERDAM, NL, vol. 689, no. 7, 1 April 2004 (2004-04-01), pages 1188 - 1193, XP004496421, ISSN: 0022-328X, DOI: 10.1016/J.JORGANCHEM.2003.10.045

## Beschreibung

Die vorliegende Erfindung betrifft die Verwendung eines Pt-Xanthen-lod-Komplexes zur Katalyse einer Hydroformylierungsreaktion.

In C. Botteghi et al., Journal of Molecular Catalysis A: Chemical 200, (2003), 147-156 wird der Einsatz von Pt(Xantphos)Cl₂ zur Hydroformylierung von 2-Tosyloxystyrol beschrieben.

In G. Petöcz et al., Journal of Organometallic Chemistry 689, (2004), 1188-1193, "Xantphos as cis- and trans-chelating ligand in square-planar platinum(II) complexes. Hydroformylation of styrene with platinum-xantphos-tin(II)chloride system" wird Xantphos als Ligand von Pt-Komplexen beschrieben.

In WO 2010/129030 A2 wird ein Verfahren zur selektiven Herstellung von Vinylester durch die Reaktion einer Carbonsäure mit Acetylen mit einem homogenen Katalysator beschrieben.

Der vorliegende Erfindung lag die Aufgabe zugrunde, die Verwendung eines Komplexes in der Hydroformylierung bereitzustellen.

Der Komplex soll hierbei bei der Katalyse von Hydroformylierungsreaktionen gegenüber dem im Stand der Technik beschriebenen Komplex Pt(Xantphos)Cl₂ eine gesteigerte Ausbeute liefern.

Diese Aufgabe wird gelöst durch die Verwendung gemäß Anspruch 1.

Verwendung eines Komplexes zur Katalyse einer Hydroformylierungsreaktion umfassen:
a) Pt;
b) einen Liganden entsprechend der Formel (I): 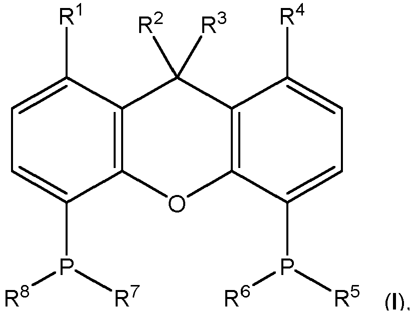 wobei R¹, R², R³, R⁴, R⁵, R⁶, R⁷, R⁸ ausgewählt sind aus: -H, -(C₁-C₁₂)-Alkyl, -(C₆-C₂₀)-Aryl; und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) einen lod-Liganden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform sind R², R³, R⁵, R⁶, R⁷, R⁸ ausgewählt aus: -(C₁-C₁₂)-Alkyl, -(C₆-C₂₀)-Aryl.

In einer Ausführungsform stehen R⁵, R⁶, R⁷, R⁸ für -(C₆-C₂₀)-Aryl.

In einer Ausführungsform stehen R⁵, R⁶, R⁷, R⁸ für -Ph
In einer Ausführungsform stehen R² und R³ für -(C₁-C₁₂)-Alkyl.

In einer Ausführungsform R² und R³ für -CH₃.

In einer Ausführungsform stehen R¹ und R⁴ für -H.

In einer Ausführungsform weist der Ligand entsprechend der Formel (**I**) die Struktur (**1**) auf:

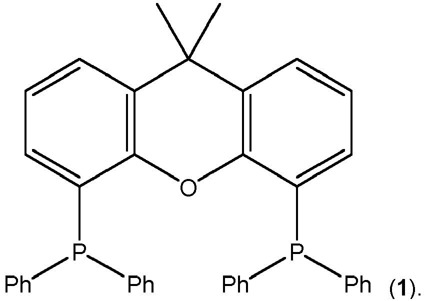

### (1): Xantphos

In einer Ausführungsform weist der Komplex genau einen Liganden entsprechend der Formel (I) auf.

In einer Ausführungsform weist der Komplex mindestens zwei lod-Liganden auf.

In einer Ausführungsform weist der Komplex genau zwei lod-Liganden auf.

In einer Ausführungsform weist der Komplex die folgenden Struktur auf: Pt(Xantphos)I₂.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und Olefin in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 20 h / 18 h bei 120 °C / 80 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Metalls

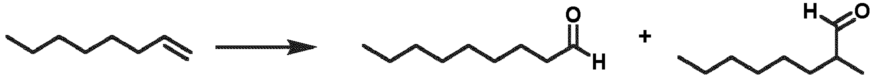

### Reaktionsbedingungen:

20 mmol 1-Octen, 0.1 mol% Metall, 2.2 Äquivalente Xantphos (1), Lösungsmittel: Toluol, p(CO/H₂): 40 bar, T: 80 °C, t: 20 h.

### Ausbeuten:

Pt(Xantphos)I₂: 99%
Pd(Xantphos)I₂: 0%

### Variation des Halogens (2-Octen)

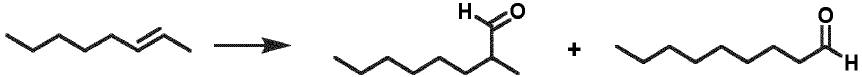

### Reaktionsbedingungen:

20 mmol 2-Octen, 1.0 mol% Pt, 1.1 Äquivalente Xantphos (1), Lösungsmittel: Toluol, p(CO/H₂): 40 bar, T: 120 °C, t: 20 h.

### Ausbeuten:

PtI₂: 99%
PtCl₂: 16%

### Variation des Halogens (1-Octen)

### Reaktionsbedingungen:

10.0 mmol 1-Octen, 0.1 mol% PtX₂, 2.2 Äquivalente Ligand, Lösungsmittel: Toluol, p(CO/H₂): 40 bar, T: 120 °C, t: 20 h.

### Ausbeuten:

| Ligand | Halogen | Ausbeute [%] |
|---|---|---|
| 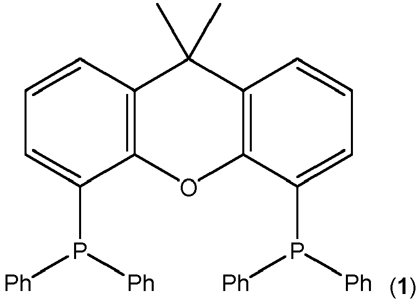 | I / Cl | 99 / 5 |

### Variation des Olefins

### Reaktionsbedingungen:

1.0 mmol Olefin, 0.5 mol% PtI₂, 2.2 äquivalente Xantphos (1), Lösungsmittel: Dichlormethan (DCM), p(CO/H₂): 40 bar, T: 80 °C, t: 18 h.

### Ausbeuten:

| Aldehyd | Ausbeute [%] |
|---|---|
| 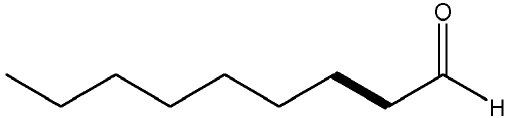 | 99 |
| 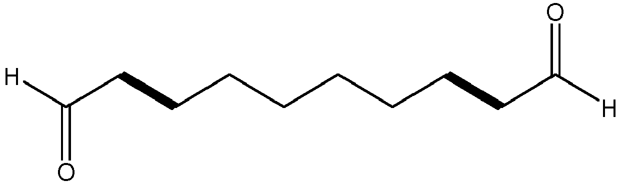 | 99 |
| 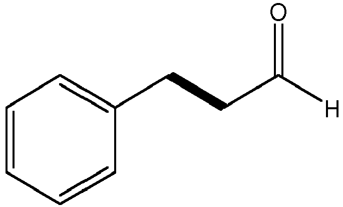 | 99 |
| 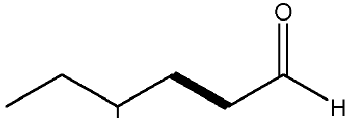 | 99 |

Die fett Markierte C-C-Bindung gibt die Lage der ehemaligen Doppelbindung, also der Doppelbindung im Olefin, an.

### Variation des Liganden und des Halogens

### Reaktionsbedingungen:

1.0 mmol 1-Octen, 0.5 mol% PtX2, 2,0 äquivalente Ligand, Lösungsmittel: Toluol, p(CO/H₂): 40 bar, T: 80 °C, t: 18 h.

### Ausbeuten:

| Ligand | Halogen | Ausbeute [%] |
|---|---|---|
| 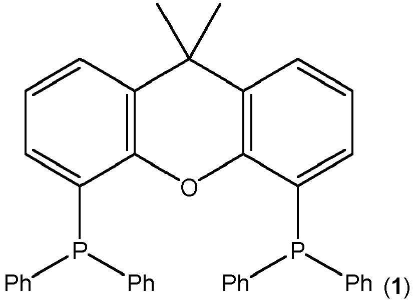 | I / Cl | 99 / 0 |
| 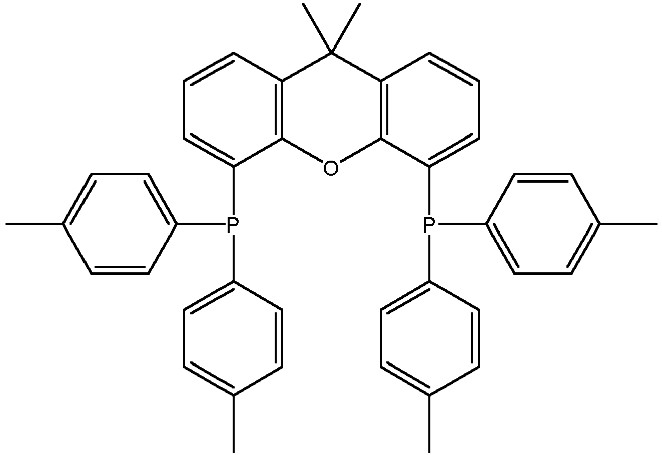 | I / Cl | 99 / 0 |
| 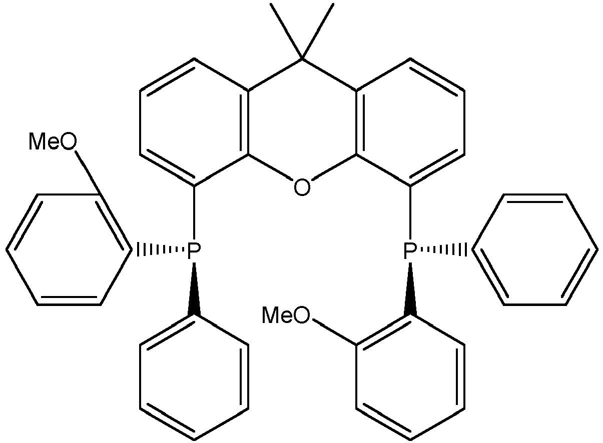 | I / Cl | 99 / < 1 |
| 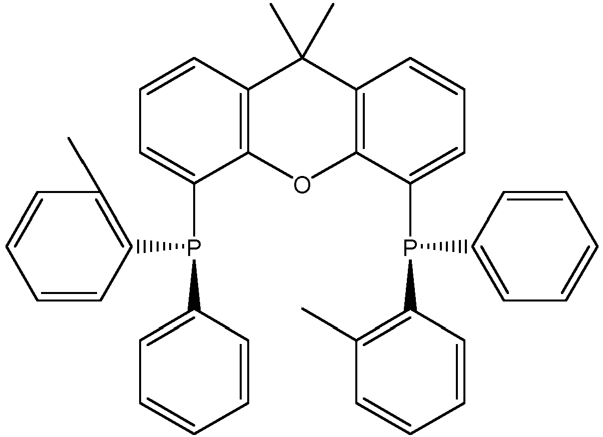 | I / Cl | 95 / 0 |

### Variation der Äquivalente und des Halogens

### Reaktionsbedingungen:

1.0 mmol 1-Octen, 1.0 mol% Pt(acac)₂, LiX (X= Halogen), 2.2 Äquivalente Xantphos (1), Lösungsmittel: Toluol, p(CO/H₂): 40 bar, T: 120 °C, t: 20 h.

| Äquivalente LiX | X | Ausbeute [%] |
|---|---|---|
| 0.5 | I | 99 |
| 1.0 | I | 99 |
| 2.0 | I | 99 |
| 4.0 | I | 99 |
| 4.0 | Cl | 0 |

Wie die Versuchsergebnisse zeigen, wird die Aufgabe durch den erfindungsgemäßen Komplex gelöst.

## Patentansprüche

1. Verwendung eines Komplexes zur Katalyse einer Hydroformylierungsreaktion umfassen:
a) Pt;
b) einen Liganden entsprechend der Formel (**I**): 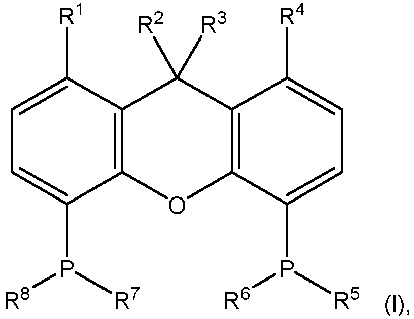 wobei R¹, R², R³, R⁴, R⁵, R⁶, R⁷, R⁸ ausgewählt sind aus: -H, -(C₁-C₁₂)-Alkyl, -(C₆-C₂₀)-Aryl; und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) einen lod-Liganden.

2. Verwendung eines Komplexes zur Katalyse einer Hydroformylierungsreaktion nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Verwendung eines Komplexes zur Katalyse einer Hydroformylierungsreaktion nach einem der Ansprüche 1 oder 2,
wobei R⁵, R⁶, R⁷, R⁸ für -(C₆-C₂₀)-Aryl stehen.

4. Verwendung eines Komplexes zur Katalyse einer Hydroformylierungsreaktion nach einem der Ansprüche 1 bis 3,
wobei R² und R³ für -(C₁-C₁₂)-Alkyl stehen.

5. Verwendung eines Komplexes zur Katalyse einer Hydroformylierungsreaktion nach einem der Ansprüche 1 bis 4,
wobei R¹ und R⁴ für -H stehen.

6. Verwendung eines Komplexes zur Katalyse einer Hydroformylierungsreaktion nach einem der Ansprüche 1 bis 5,
wobei der Ligand entsprechend der Formel (**I**) die Struktur (**1**) aufweist:

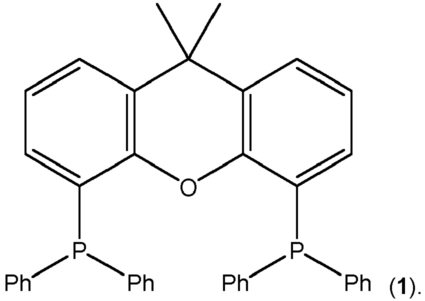

7. Verwendung eines Komplexes zur Katalyse einer Hydroformylierungsreaktion nach einem der Ansprüche 1 bis 6,
wobei der Komplex genau einen Liganden entsprechend der Formel (**I**) aufweist.

8. Verwendung eines Komplexes zur Katalyse einer Hydroformylierungsreaktion nach einem der Ansprüche 1 bis 7,
wobei der Komplex mindestens zwei lod-Liganden aufweist.

9. Verwendung eines Komplexes zur Katalyse einer Hydroformylierungsreaktion nach einem der Ansprüche 1 bis 8,
wobei der Komplex genau zwei lod-Liganden aufweist.

10. Verwendung eines Komplexes zur Katalyse einer Hydroformylierungsreaktion nach einem der Ansprüche 1 bis 9,
wobei der Komplex die folgenden Struktur aufweist: Pt(Xantphos)I₂.

## Claims

1. Use of a complex for catalysis of a hydroformylation reaction comprising:
a) Pt;
b) a ligand corresponding to formula (I): 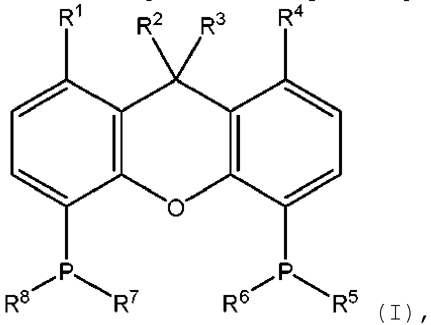 where R¹, R², R³, R⁴, R⁵, R⁶, R⁷, R⁸ are selected from: -H, -(C₁-C₁₂)-alkyl, -(C₆-C₂₀)-aryl; and, if R¹, R², R³, R⁴, R⁵, R⁶, R⁷, R⁸ are -(C₆-C₂₀)-aryl, the aryl ring may have substituents selected from: -(C₁-C₁₂)-alkyl, -O-(C₁-C₁₂)-alkyl;
c) an iodine ligand.

2. Use of a complex for catalysis of a hydroformylation reaction according to Claim 1,
wherein R², R³, R⁵, R⁶, R⁷, R⁸ are selected from: -(C₁-C₁₂)-alkyl, -(C₆-C₂₀)-aryl.

3. Use of a complex for catalysis of a hydroformylation reaction according to either of Claims 1 and 2,
wherein R⁵, R⁶, R⁷, R⁸ are -(C₆-C₂₀)-aryl.

4. Use of a complex for catalysis of a hydroformylation reaction according to any of Claims 1 to 3,
wherein R² and R³ are -(C₁-C₁₂)-alkyl .

5. Use of a complex for catalysis of a hydroformylation reaction according to any of Claims 1 to 4,
wherein R¹ and R⁴ are -H.

6. Use of a complex for catalysis of a hydroformylation reaction according to any of Claims 1 to 5,
wherein the ligand corresponding to formula (I) has the structure (1):

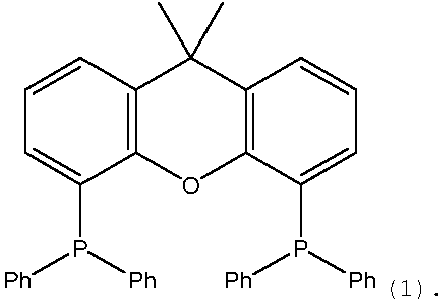

7. Use of a complex for catalysis of a hydroformylation reaction according to any of Claims 1 to 6,
wherein the complex has exactly one ligand corresponding to formula (I).

8. Use of a complex for catalysis of a hydroformylation reaction according to any of Claims 1 to 7,
wherein the complex has at least two iodine ligands.

9. Use of a complex for catalysis of a hydroformylation reaction according to any of Claims 1 to 8,
wherein the complex has exactly two iodine ligands.

10. Use of a complex for catalysis of a hydroformylation reaction according to any of Claims 1 to 9,
wherein the complex has the following structure: Pt(Xantphos)I₂.

## Revendications

1. Utilisation d'un complexe pour la catalyse d'une réaction d'hydroformylation, comprenant :
a) du Pt ;
b) un ligand selon la formule (I) : 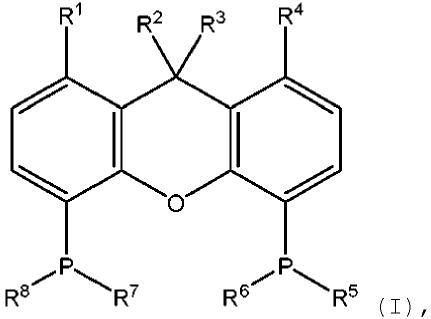 dans laquelle R¹, R², R³, R⁴, R⁵, R⁶, R⁷, R⁸ sont choisis parmi : -H,-(C₁-C₁₂)-alkyle, -(C₆-C₂₀)-aryle ; et pour le cas où R¹, R², R³, R⁴, R⁵, R⁶, R⁷, R⁸ représentent -(C₆-C₂₀)-aryle, le cycle aryle peut présenter des substituants qui sont choisis parmi : -(C₁-C₁₂)-alkyle, -O-(C₁-C₁₂)-alkyle ;
c) un ligand iode.

2. Utilisation d'un complexe pour la catalyse d'une réaction d'hydroformylation selon la revendication 1,
R², R³, R⁵, R⁶, R⁷, R⁸ étant choisis parmi : -(C₁-C₁₂)-alkyle, -(C₆-C₂₀)-aryle.

3. Utilisation d'un complexe pour la catalyse d'une réaction d'hydroformylation selon l'une des revendications 1 ou 2,
R⁵, R⁶, R⁷, R⁸ représentant -(C₆-C₂₀)-aryle.

4. Utilisation d'un complexe pour la catalyse d'une réaction d'hydroformylation selon l'une des revendications 1 à 3,
R² et R³ représentant -(C₁-C₁₂)-alkyle.

5. Utilisation d'un complexe pour la catalyse d'une réaction d'hydroformylation selon l'une des revendications 1 à 4,
R¹ et R⁴ représentant -H.

6. Utilisation d'un complexe pour la catalyse d'une réaction d'hydroformylation selon l'une des revendications 1 à 5,
le ligand selon la formule (I) présentant la structure (1) :

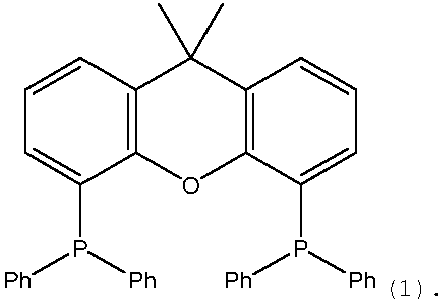

7. Utilisation d'un complexe pour la catalyse d'une réaction d'hydroformylation selon l'une des revendications 1 à 6,
le complexe présentant exactement un ligand selon la formule (I).

8. Utilisation d'un complexe pour la catalyse d'une réaction d'hydroformylation selon l'une des revendications 1 à 7,
le complexe présentant au moins deux ligands iode.

9. Utilisation d'un complexe pour la catalyse d'une réaction d'hydroformylation selon l'une des revendications 1 à 8,
le complexe présentant exactement deux ligands iode.

10. Utilisation d'un complexe pour la catalyse d'une réaction d'hydroformylation selon l'une des revendications 1 à 9,
le complexe présentant la structure suivante : Pt(Xantphos)I₂.
